# EUROPEAN PATENT APPLICATION

(11) **EP 3 395 953 A1**
(43) Date of publication of application: **31.10.2018**
(21) Application number: 16878314.0
(22) Date of filing: 02.12.2016
(51) Int. Cl.: C12P 19/26

(54) **METHOD FOR PRODUCING MACROMOLECULAR HYALURONIC ACID**

(30) Priority: 24.12.2015 JP 2015251588
(71) Applicant: Kikkoman Corporation, Noda-shi, Chiba 278-8601 (JP)
(72) Inventor: NOZAWA, Takashi, Noda-shi Chiba 278-8601 (JP); NISHIMOTO, Kazunori, Noda-shi Chiba 278-8601 (JP); MAEDA, Yasukazu, Noda-shi Chiba 278-8601 (JP)
(74) Representative: Zacco Sweden AB
(86) International application number: PCT/JP2016/085870
(87) International publication number: WO 2017/110421

(57) **Abstract**

The present invention provides a method for producing a high molecular weight hyaluronic acid. More specifically, the present invention provides a method for producing hyaluronic acid comprising a step of culturing a microorganism capable of producing hyaluronic acid in a culture medium having a salt concentration of from 2 w/v% or more and 5 w/v% or less, or 0.2 mol/L or more and 0.9 mol/L or less.

## Description

### Technical Field

The present invention relates to a method for producing high molecular weight hyaluronic acid, high molecular weight hyaluronic acid obtained using the method, and pharmaceuticals, food or drink, and compositions containing the high molecular weight hyaluronic acid.

### Background Art

Hyaluronic acid is a linear polysaccharide consisting of a repeating structure of disaccharides composed of N-acetyl-D-glucosamine and D-glucuronic acid, and is a material that exhibits high moisture retention, high viscoelasticity, high lubricity, and so on. Because of these characteristics, hyaluronic acid is used in a number of applications, ranging from a humectant in cosmetic products to an arthropathic remedy, an ophthalmic surgical adjuvant and the like in a medical field. Interest in the functions of hyaluronic acid has grown in recent years, and a number of supplements and luxury foods containing hyaluronic acid are now available in the market. Because hyaluronic acid is known to have different physiological actions depending on its molecular weight, hyaluronic acid with different average molecular weights is required depending on the application.

The hyaluronic acid used in food products and cosmetic products is generally obtained as an extract from cockscomb or by culturing a microorganism capable of producing hyaluronic acid such as *Streptococcus* in a culture medium and then purifying the product. A method has been proposed for producing hyaluronic acid of a specific molecular weight in which hyaluronic acid having a molecular weight exceeding 6 x 10⁶ Da is obtained by culturing mutant strains of *Streptococcus equi* (see, for example, Patent Document 1).

### Prior Art Documents

### Patent Document

Patent Document 1: Japanese Patent No. 3720361

### Summary of the Invention

Problem to be Solved by the Invention

It is the primary object of the present invention to provide a method for obtaining high molecular weight hyaluronic acid.

### Means for Solving the Problem

As a result of extensive research, the present inventors discovered that the object of the present invention could be achieved by controlling the salt concentration in the culture medium used to culture the microorganism producing hyaluronic acid. The present invention is a product of this discovery.

Specifically, the present invention is related to the following.
[1] A method for producing hyaluronic acid comprising a step of culturing a microorganism capable of producing hyaluronic acid in a culture medium having a salt concentration of 2 w/v% or more and 5 w/v% or less.
[2] The method according to [1], wherein the salt concentration in the culture medium is 2 w/v% or more and 4 w/v% or less.
[3] A method for producing hyaluronic acid comprising a step of culturing a microorganism capable of producing hyaluronic acid in a culture medium having a salt concentration of 0.2 mol/L or more and 0.9 mol/L or less.
[4] The method according to any one of [1] to [3], wherein the microorganism capable of producing hyaluronic acid is selected from a group consisting of *Streptococcus equi subsp. zooepidemicus, Streptococcus equi subsp. equi,* and *Streptococcus pyogenes.*
[5] The method according to [4], wherein the microorganism capable of producing hyaluronic acid is *Streptococcus equi subsp. zooepidemicus.*
[6] The method according to any one of [1] to [5], wherein the salt is selected from sodium chloride and sodium sulfate.
[7] The method according to any one of [1] to [6], wherein the molecular weight of the produced hyaluronic acid is 7,000,000 or higher.

### Effect of the Invention

According to the present invention, high molecular weight hyaluronic acid can be obtained.

### Embodiment for carrying out the Invention

### (1) Microorganism capable of producing hyaluronic acid

In the present invention, any microorganism that produces hyaluronic acid may be used as a microorganism capable of producing hyaluronic acid without limitation. These include microorganisms existing in nature and microorganisms that have been genetically modified so as to be able to produce hyaluronic acid. In one aspect of the present invention, the microorganism capable of producing hyaluronic acid is preferably a microorganism belonging to the genus *Streptococcus.* Examples of microorganisms belonging to the genus *Streptococcus* include *Streptococcus equi subsp. zooepidemicus, Streptococcus equi subsp. equi,* and *Streptococcus pyogenes.* Among these, *Streptococcus equi subsp. zooepidemicus* is especially preferred.

### (2) Salt concentration of culture medium

In the present invention, the concentration of salt in the culture medium is adjusted when a microorganism capable of producing hyaluronic acid is cultured in a culture medium to produce hyaluronic acid.

In one aspect of the present invention, the concentration of salt in the culture medium depends on the type of microorganism, but is greater than 1 w/v%, preferably 2 w/v% or more, and more preferably 3 w/v% or more from the standpoint of the ability of producing high molecular weight hyaluronic acid. From the standpoint of easier microorganism growth, the concentration is 6 w/v% or less, preferably 5 w/v% or less, and more preferably 4 w/v% or less. For example, a microorganism can be cultured in a culture medium having a salt concentration of greater than 1 w/v% and 6 w/v% or less, preferably 2 w/v% or more and 5 w/v% or less, more preferably 2 w/v% or more and 4 w/v% or less, and still more preferably 3 w/v% or more and4 w/v% or less.

In another aspect of the present invention, the concentration of salt in the culture medium depends on the type of microorganism, but is 0.2 mol/L or more, preferably 0.3 mol/L or more, more preferably 0.4 mol/L or more, and still more preferably 0.5 mol/L or more from the standpoint of the ability of producing high molecular weight hyaluronic acid. From the standpoint of easier microorganism growth, the concentration is 1.0 mol/L or less, preferably 0.9 mol/L or less, more preferably 0.8 mol/L or less, and still more preferably 0.7 mol/L or less. For example, a microorganism can be cultured in a culture medium having a salt concentration of0.2 mol/L or more and 1.0 mol/L or less, preferably 0.2 mol/L or more and 0.9 mol/L or less, more preferably 0.3 mol/L or more and 0.8 mol/L or less, even more preferably 0.4 mol/L or more and 0.8 mol/L or less, and still more preferably 0.5 mol/L or more and 0.7 mol/L or less.

Higher molecular weight hyaluronic acid can be obtained by culturing a microorganism at the aforementioned salt concentrations. While not bound by theory, the salt stress response at high salt concentrations appears to cause microorganisms to produce higher molecular weight hyaluronic acid.

The salt used to adjust the salt concentration can be any salt so long as the microorganisms are capable of growing. Examples include sodium chloride and sodium sulfate. Sodium chloride is preferred.

### (3) Culture medium

With the exception of the concentration of salt, the culture medium can be any culture medium known in the art used to culture the various types of microorganisms. For example, a culture medium can be used in which the carbon source is a monosaccharide such as glucose or fructose, a disaccharide such as lactose, sucrose or maltose, or an oligosaccharide; the nitrogen source is a polypeptone; and the organic nitrogen source is a yeast extract. If necessary, free amino acids such as arginine, glutamic acid and glutamine, as well as vitamins may be added. Also, the pH of the culture medium may be adjusted depending on the type of microorganism. For example, the pH can be from 5.0 to 8.5, and preferably from 6.5 to 7.5.

### (4) Culturing conditions

The culturing conditions can be selected arbitrary depending on the type of microorganism, and technique known in the art can be used such as shake culturing and aeration-stirring culturing. The temperature of the culture medium may be adjusted within a range from 25 to 42°C, preferably from 30 to 40°C, and more preferably from 32 to 38°C. If necessary, the microorganism can be cultured aerobically. There are no particular restrictions on the incubation time. This can be set based on the amount of culture medium and the desired amount of hyaluronic acid.

### (5) Isolation and purification of hyaluronic acid

The hyaluronic acid produced by the microorganism can be isolated and purified depending on the intended application using any method known in the art. For example, cells and culture-derived insoluble components can be removed to obtain roughly purified hyaluronic acid using heat sterilization, various filtration, centrifugation or the like, and the roughly purified hyaluronic acid can then be added to a food or drink or blended into a cosmetic product. Alternatively, highly pure hyaluronic acid can be obtained using any hyaluronic acid purification method common in the art such as solvent precipitation using ethanol, and the highly pure hyaluronic acid can then be used in a food or drink, cosmetics, pharmaceuticals and the like. A salt of hyaluronic acid such as a sodium salt can also be isolated and purified using any method known in the art.

Compared to hyaluronic acid obtained from the same microorganism using a method other than the method of the present invention, hyaluronic acid produced using the method of the present invention has a higher average molecular weight, for example, an average molecular weight of 6,500,000 or higher, preferably 7,000,000 or higher. The average molecular weight can be measured using any method known in the art, such as the intrinsic viscosity method or the SEC-MALS method. For example, the average molecular weight can be measured referring to the method described in the examples section below.

### Examples

### [Culturing of Streptococcus equi subsp. zooepidemicus ATCC43079]

Sodium chloride was added to a culture medium comprising 1.0% glucose, 1.5% yeast extract, and 0.5% soy peptone in an amount of 0, 1, 2, 3, or 4% (that is, 0, 0.17, 0.34, 0.51, or 0.68 mol/L). In the examples and comparative example, "%" means "w/v%" unless otherwise indicated. After adjusting each culture medium to a pH from 6.9 to 7.0 using a 3 mol/L sodium hydroxide aqueous solution, 50 mL of each culture medium was added to a 200 mL Erlenmeyer flask with a baffle containing 0.25 g of weighed calcium carbonate, and the flask was sterilized in an autoclave. Each culture was inoculated with *Streptococcus equi subsp. zooepidemicus* ATCC43079 and shake-cultured at 33°C and 120 rpm. The incubation times are shown in Table 1. The incubation time was set so as to take into account the fact that the growth rate of the microorganism declines as the salt concentration rises.

### [Measuring the molecular weight and concentration of hyaluronic acid]

The molecular weight and concentration of hyaluronic acid produced in each culture medium were measured in the following manner. Each culture medium was diluted using a 0.2 mol/L sodium nitrate aqueous solution to adjust the concentration of hyaluronic acid to 0.02% or less, and the culture medium was passed through a filter with a pore size of 0.2 µm to obtain a test solution. The resulting solution was analyzed using a system combining size exclusion chromatography (SEC) and multi-angle light scattering (MALS) under the following conditions. Note that ASTRAV (Wyatt Technology Corporation) was used as the analysis software. The results are shown in Table 1. Higher molecular weight hyaluronic acid was obtained as the salt concentration in the culture medium rose.
(i) Equipment
   SEC system: Shodex GPC-101 (Showa Denko Co., Ltd.)
(ii) Analytical conditions
   Column: Shodex OHpak SB-807HQ
   Column bath temperature: 40°C
   Eluent: 0.2 mol/L sodium nitrate aqueous solution
   Flow rate: 0.4 mL/min
   Amount of sample loaded: 100 µL
   Light scattering meter: Dawn Heleos-II (Wyatt Technology Corporation)
   Analysis time: 40 min

**[Table 1]**

| NaCl (%) | NaCl (mol/L) | Incubation Time (hr) | Mw (MDa) | Concentration (%) |
|---|---|---|---|---|
| 0 | 0 | 21 | 5.17 | 0.090 |
| 1 | 0.17 | 24 | 5.57 | 0.077 |
| 2 | 0.34 | 27 | 7.17 | 0.043 |
| 3 | 0.51 | 30 | 8.32 | 0.021 |
| 4 | 0.68 | 33 | 9.68 | 0.013 |

### < Comparative Example >

A culture medium comprising 1.0% glucose, 1.5% yeast extract, 0.5% soy peptone, and 0.2% sodium chloride (that is, 0.034 mol/L) was adjusted to a pH from 6.9 to 7.0 using a 3 mol/L sodium hydroxide aqueous solution. Fifty (50) mL of this culture medium was then added to a 200 mL Erlenmeyer flask with a baffle containing 0.25 g of weighed calcium carbonate, and the flask was sterilized in an autoclave. The culture was inoculated with *Streptococcus equi subsp. zooepidemicus* ATCC43079 and shake-cultured at 33°C and 120 rpm. The molecular weight and concentration of hyaluronic acid produced in the culture medium were measured in the same manner as the example. The incubation times and measurement results are shown in Table 2. When the salt concentration in the culture medium was low, high molecular weight hyaluronic acid could not be obtained even when culturing was continued.

**[Table 2]**

| Incubation Time (hr) | Mw (MDa) | Concentration (%) |
|---|---|---|
| 6 | 4.39 | 0.003 |
| 9 | 5.68 | 0.005 |
| 12 | 5.16 | 0.025 |
| 15 | 4.56 | 0.074 |
| 18 | 6.28 | 0.070 |
| 21 | 5.86 | 0.076 |
| 24 | 6.35 | 0.068 |

### Industrial Applicability

The present invention has industrial applicability in that it provides a simple method for producing high molecular weight hyaluronic acid. The present invention is useful in the fields of pharmaceuticals, cosmetics, and food products.

The present application claims priority on the basis of Japanese Patent Application No. 2015-251588 filed on December 24, 2015, the contents of which have been incorporated herein by reference.

## Claims

1. A method for producing hyaluronic acid comprising a step of culturing a microorganism capable of producing hyaluronic acid in a culture medium having a salt concentration of 2 w/v% or more and 5 w/v% or less.

2. The method according to claim 1, wherein the salt concentration in the culture medium is 2 w/v% or more and 4 w/v% or less.

3. A method for producing hyaluronic acid comprising a step of culturing a microorganism capable of producing hyaluronic acid in a culture medium having a salt concentration of 0.2 mol/L or more and 0.9 mol/L or less.

4. The method according to any one of claims 1 to 3, wherein the microorganism capable of producing hyaluronic acid is selected from a group consisting of *Streptococcus equi subsp. zooepidemicus, Streptococcus equi subsp. equi,* and *Streptococcus pyogenes.*

5. The method according to claim 4, wherein the microorganism capable of producing hyaluronic acid is *Streptococcus equi subsp. zooepidemicus.*

6. The method according to any one of claims 1 to 5, wherein the salt is selected from sodium chloride and sodium sulfate.

7. The method according to any one of claims 1 to 6, wherein the molecular weight of the produced hyaluronic acid is 7,000,000 or higher.
